# EUROPEAN PATENT SPECIFICATION

(11) **EP 1 572 636 B1**
(45) Date of publication and mention of the grant of the patent: **18.07.2007**
(21) Application number: 03813507.5
(22) Date of filing: 12.12.2003
(51) Int. Cl.: C07C 321/28, A61K 8/46, C11B 9/00, A23L 1/226

(54) **ALKYLSULFANYL-BENZENES AS FRAGRANCE COMPOUNDS**
ALKYLSULFANYLBENZOLE ALS DUFTSTOFFE
ALKYLSULFANYL-BENZENES UTILISES EN TANT QUE COMPOSES DE PARFUM

(30) Priority: 19.12.2002 GB 0229453
(43) Date of publication of application: 14.09.2005
(73) Proprietor: Givaudan SA, 1214 Vernier-Genève (CH)
(72) Inventor: GOEKE, Andreas, Shanghai 201203 (CN)
(74) Representative: McStea, John Anthony
(86) International application number: PCT/CH2003/000814
(87) International publication number: WO 2004/056765

(56) References cited:
- EP-A- 1 264 547
- US-A- 4 200 660
- DATABASE CA [Online] CHEMICAL ABSTRACTS SERVICE, COLUMBUS, OHIO, US; OGAWA, HAZUKI ET AL: "Methylthioalkylbenzene compounds as fragrances or flavors" retrieved from STN Database accession no. 112:104604 XP002273427 & JP 01 161092 A (LION CORP., JAPAN;TOYOTAMA PERFUMERY CO., LTD.) 23 June 1989 (1989-06-23)
- DATABASE CROSSFIRE BEILSTEIN [Online] Beilstein Institut zur Förderung der Chemischen Wissenschaften, Frankfurt am Main, DE; Database accession no. BRN 3234001 XP002273428 & BUU-HOI;HOAN: J.ORG.CHEM, vol. 17, 1952, pages 350-356,
- DATABASE CROSSFIRE BEILSTEIN [Online] Beilstein Institut zur Förderung der Chemischen Wissenschaften, Frankfurt am Main, DE; Database accession no. BRN 3233636 XP002273429 & HUGHES;THOMPSON: J.PROC.R.SOC.N.S.W., vol. 82, 1948, page 262

## Description

This invention relates to alkylsulfanyl-benzenes, and in particular those having spicy and anisic odour notes, a method of producing the same and to flavour and fragrance compositions containing one or more of these compounds.

Compounds having spicy and anisic odour notes are of interest in the flavour and fragrance industry.

However, certain molecules, e.g. eugenol and anethole, despite having these interesting odour notes have the disadvantage that they discolour on storage, and therefore their use is usually limited to certain applications where discoloration is not a problem or can be masked.

Structural modification of these molecules, however, either results in a failure to retain the spicy and anisic odourant properties or the odourant properties are retained but they are far less intense and rich.

Accordingly, there remains a need to provide molecules that do not possess the disadvantages of the prior art molecules but which retain their spicy and anisic odour notes and are diffusive and substantive.

It has now be found that certain compounds can be developed that are stable to discolouration and yet are substantive, diffusive and possess the desirable spicy and anisic odour notes.

Accordingly, the invention provides in one of its aspects the use of compound of formula I as flavour or fragrance 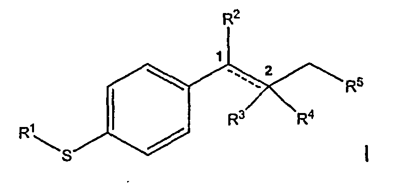 wherein
i) the bond between C₁ and C₂ is a single bond;
   R¹ is methyl, ethyl, *i*-propyl, *n*-propyl;
   R² and R³ are independently hydrogen or methyl; or
   R² and R³ taken together is a divalent radical (CH₂)ₙ, C(CH₃)₂, or CH(CH₃) which forms a cycloalkane ring together with the carbon atoms to which it is attached;
   R⁴ and R⁵ are independently hydrogen or methyl; or
   R⁴ and R⁵ taken together is a divalent radical (CH₂)ₙ, (CH₂)ₙ₋₁CH(CH₃)₂, or (CH₂)ₙ₋₁CH(CH₃) which forms a cycloalkane ring together with the carbon atoms to which it is attached;
   n is an integer of 1, 2, 3, or 4; and
   wherein at least one cycloalkane ring is present; or
ii) the bond between C₁ and C₂ together with the dotted line represents a double bond;
   R¹ is methyl, ethyl, *i*-propyl, n-propyl;
   R² is hydrogen or methyl;
   R³ and R⁴ together is hydrogen or methyl; and
   R⁵ is hydrogen, methyl, ethyl, *i*-propyl, *n*-propyl, *n*-butyl, *tert-*butyl or sec-butyl.

The compounds of formula I may comprise one or more a chiral centres and as such may exist as a mixture of stereoisomers, or they may be resolved as isomerically pure forms. Resolving stereoisomers adds to the complexity of manufacture and purification of these compounds, and so it is preferred to use the compounds as mixtures of their stereoisomers simply for economic reasons. However, if it is desired to prepare individual stereoisomers, this may be achieved according to methodology known in the art, e.g. preparative HPLC and GC or by stereoselective syntheses.

Particularly preferred compounds of formula I wherein the bond between C₁ and C₂ is a single bond are 1-cyclopropylmethyl-4-methylsulfanyl-benzene, 1-cyclobutylmethyl-4-methylsulfanyl-benzene, 1-cyclopentylmethyl-4-methylsulfanyl-benzene and 1-cyclohexylmethyl-4-methylsulfanyl-benzene.

Particularly preferred compound of formula I wherein the bond between C₁ and C₂ together with the dotted line is a double bond is 1-hex-1-enyl-4-methylsulfanyl-benzene.

Compounds of formula I may be used alone or as a mixture to form a fragrance composition, which composition forms another aspect of the present invention. In addition, the compounds may be used in combination with other known flavourant or odourant molecules selected from the extensive range of natural and synthetic molecules currently available and/or in admixture with one or more ingredients or excipients conventionally used in conjugation with odourants or flavourants in fragrance or flavour compositions.

Compounds of formula I may be combined with a wide range of known odorants to create interesting odor notes. The following non-limiting list comprises examples of known odourant molecules which may be combined with the compounds of the present invention:
natural products: tree moss absolute, basil oil, tropical fruit oils (such as bergamot oil, mandarin oil, etc.), mastix absolute, myrtle oil, palmarosa oil, patchouli oil, petitgrain oil, wormwood oil, lavender oil, rose oil, jasmin oil, ylang-ylang oil.
alcohols: farnesol, geraniol, linalool, nerol, phenylethyl alcohol, rhodinol, cinnamic alcohol, (Z)-hex-3-en-1-ol, menthol, α-terpineol.
aldehydes: citral, α-hexyl cinnamaldehyde, Lilial, methylionone, verbenone, nootkatone, geranylacetone.
esters: allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, methyl dihydrojasmonate, styralyl propionate, vetineryl acetate, benzyl acetate, geranyl acetate.
lactones: γ-undecalactone, δ-decalactone, pentadecanolide, 12-oxahexadecanolide.
acetals: Viridine (phenylacetaldehyde dimethylacetal).
other components often used in perfumery: indole, p-mentha-8-thiol-3-one, methyleugenol, eugenol, anethol.

The compounds of the present invention may be used neat and simply admixed to form compositions. Alternatively or additionally however, they may be entrapped with entrapment materials, for example, polymers, capsules, microcapsules and nanocapsules, liposomes, film formers, absorbents such as carbon or zeolites, cyclic oligosaccharides and mixtures thereof, or they may be chemically bound to substrates which are adapted to release the compound of formula I upon application of an exogenous stimulus such as light; enzyme, or the like, and these entrapped forms may be used in compositions of the present invention.

The compositions may optionally comprise other excipients well known in the art, including anti-foaming agents, anti-oxidant agents, binders, colourants, diluents, disintegrants, emulsifiers, enzymes, fats, flavour-enhancers, flavouring agents, gums, lubricants, polysaccharides, preservatives, proteins, solubilisers, solvents, stabilisers, sugar-derivatives, surfactants, sweetening agents, vitamins, waxes, and the like. Solvents which may be used are known to those skilled in the art and include e.g. ethanol, ethylene glycol, propylene glycol, glycerol, triacetin, diethyl phthalate and dimethyl phthalate.

Examples of absorbents, entrapment materials, excipients, diluents or solvents for flavour or fragrance applications may be found e.g. in _{"}Perfume and Flavor Materials of Natural Origin", S. Arctander, Ed., Elizabeth, N.J., 1960; in "Perfume and Flavor Chemicals", S. Arctander, Ed., Vol. I & II, Allured Publishing Corporation, Carol Stream, USA, 1994; in "Flavourings", E. Ziegler and H. Ziegler (ed.), Wiley-VCH Weinheim, 1998, and "CTFA Cosmetic Ingredient Handbook", J.M. Nikitakis (ed.), 1st ed., The Cosmetic, Toiletry and Fragrance Association, Inc., Washington, 1988.

The compounds of the present invention may be used in fragrance applications, e.g. in any field of fine and functional perfumery, such as perfumes, household products, laundry products, body care products and cosmetics. The high diffusion and substantivity of compounds according to the present invention are well perceived on fabrics washed with detergent or treated with a softener comprising them. The typical spicy anisic odour is already perceived on wet fabric and lingers for long periods, e.g. 2 - 4 days on dry fabric.

The compounds of formula I may be used In flavour applications and are useful in modifying for example, spicy flavours and seasonings for condiments and meats. They may be used in aromatic, herbal and spicy flavourings, heavy fruit flavours (e.g. raisin, prune) and in flavours for root beer. The compounds are also well suited for mouthwash applications.

In flavourant applications, the compounds of the formula I may be present in compositions in amounts ranging from 0.001 to 1000 mg/kg, more preferably from 0.05 to 500 mg/kg.

When used in fragrance applications, compounds of the formula I can be employed in widely ranging amounts depending upon the specific application, for example, from 0.001 to 10 weight percent. One application may be a fabric softener comprising 0.001 to 0.05 weight percent. Another application may be an alcoholic solution comprising 0.1 to 10 weight percent. The preferred concentrations vary between 0.1 and 5 weight percent. However, the values are not limiting on the present invention, since the experienced perfumer may also achieve effects with even lower concentrations or may create novel accords with even higher amounts.

Whereas some compounds have been described in the literature, others have not, and are novel. Thus, in an other aspect of the invention, there is provided a compound of formula I 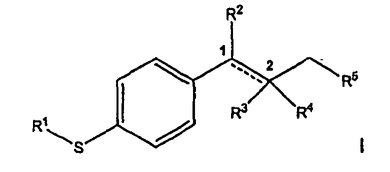 wherein R¹, R², R³, R⁴ and R⁵ have the same meaning as given above with the proviso that if the bond between C₁ and C₂ together with the dotted line represents a double bond, R¹ is ethyl, R² is hydrogen, and R³ and R⁴ together is hydrogen, then R⁶ is not hydrogen; or

if the bond between C₁ and C₂ together with the dotted line represents a double bond, R¹ is methyl, R² is hydrogen, and R³ and R⁴ together is hydrogen, then R⁵ is not hydrogen or methyl.

The compounds of formula I may be synthesised from commonly-available starting materials and reagents according to synthetic protocols known in the art. Benzene-(4-alkylsulfanyl-cycloalkyl) compounds of formula I (i.e. R² and R³ forms a cycloalkane ring together with the carbon atoms to which they are attached) may be synthesised from the corresponding 4-alkylsulfanyl-phenyl carbonyl compound, e.g. 4-methylsulfanyl-benzaldehyde, under Wittig reaction conditions, followed by cycloalkylation of the intermediate alkylsulfanyl alkenyl benzene, e.g. 1-methylsulfanyl-4-propenyl-benzene, under Simmons-Smith reaction conditions, well known in the art.

Benzene-(4-alkylsulfanyl-cycloalkyl-methyl) compounds of formula I (i.e. R⁴ and R⁵ forms a cycloalkane ring together with the carbon atoms to which they are attached) may be synthesised from the corresponding 4-alkylsulfanyl-benzene, e.g. thioanisol (C₆H₅SCH₃), and the corresponding carboxylic acid chloride, e.g. cyclopropanecarboxylic acid chloride, followed by reduction of the intermediate ketone, e.g. cyclopropyl-(4-methylsulfanyl-phenyl)-methanone, under Wolff-Kishner reaction conditions well known in the art.

Further particulars as to reaction conditions are provided in the examples.

### Example 1: 1-Prop-1-enyl-4-methylsuffanyl-benzene

To a cooled suspension (-10°C) of ethyl-triphenyl-phosphonium bromide (14.63g, 39.4 mmol) in THF was added BuLi (1.6 M solution in hexane, 24.6 ml). The mixture was stirred for 10 min and was then cooled to -78°C. 4-(Methylthio)-benzaldehyde (5.00g, 32.3 mmol) was added dropwise, the mixture was stirred for further 10 min and was then poured into a sat. solution of NH₄Cl. The mixture was diluted with pentane and the organic phase was separated, washed with water and brine and dried (MgSO₄). The residue was distilled bulb-to-bulb to yield 1-prop-1-enyl-4-methylsulfanyl-benzene (3.22g, 60%, mixture of E/Z isomers) as a colorless oil.

¹H-NMR (CDCl₃, 400MHz): 7.24-7.15 (m, 4H), 6.36-6.14/5.79-5.71 (2m, 2H), 2.46/2.44 (2s, 3H), 1.89-1.84 (m, 3H) ppm. MS (EI): 164 (M⁺, 100), 149 (14), 117 (69), 115 (58), 91 (12), 77 (8), 63 (9), 51 (5). IR (atr): 3019w, 2919w, 1592w, 1492s, 1435m, 1092m, 964s, 836s, 780s cm⁻¹.

Odor description: anisic, minty, anethole

### Example 2: 1-Hex-1-enyl-4-methylsulfanyl-benzene

Synthesized analogously to the procedure of Example 1.

¹H-NMR (CDCl₃, 400MHz): 7.24-7.14 (m, 4H), 6.35-6.12/5.66-5.59 (2m, 2H), 2.44/2.43 (2s, 3H), 2.35-2.16 (m, 2H), 1.47-1.29 (m, 4H), 0.91/0.89 (2t, J = 7.2 Hz, 3H) ppm. GC/MS (EI, major isomer): 206 (M⁺, 80), 163 (100), 150 (21), 135 (14), 116 (92), 91 (9), 77 (5), 63 (4). IR (atr): 2956m, 2922m, 2857m, 1493s, 1436m, 1094m, 965s, 838m, 801 m cm⁻¹.

Odor description: sassafras, fruity, pear, agrestic

### Example 3: 1-Cyclobutylmethyl-4-methylsulfanyl-benzene

### a) Cyclobutyl-(4-methylsulfanyl-phenyl)-methanone

Cyclobutane carboxylic acid chloride (4.50g, 38 mmol) dissolved in dichloroethane (10 ml) was added dropwise to a cold (-10°C) suspension of AlCl₃ (4.81g, 38 mmol) in dichloroethane (20 ml). To this solution was added methylsulfanyl-benzene (4.49g, 36 mmol) at such a rate that the temperature did not exceed 10°C. After the mixture was stirred for 45 min in an ice bath, it was poured on water and extracted with dichloroethane. The organic phase was separated, washed with water and brine, dried (MgSO₄) and concentrated in vacuo to yield a crystalline solid (6.78g, 91%).

¹H-NMR (CDCl₃, 400MHz): 7.81-7.79 (m, J = 6.8 Hz, 2H), 7.27-7.24 (m, J = 6.8 Hz, 2H), 3.95 (quint., J = 8.5 Hz, 1 H), 2.51 (s, 3H), 2.47-2.23 (m, 4H), 2.13-2.03 (m, 1H), 1.95-1.86 (m, H) ppm. GC/MS (EI): 206 (M⁺, 18), 178 (2), 151 (100), 123 (7), 108 (8), 79 (5), 45 (6).

### b) 1-Cyclobutylmethyl-4-methylsulfanyl-benzene

A suspension of crude cyclobutyl-(4-methylsulfanyl-phenyl)-methanone (6.78g, 33 mmol), K₂CO₃ (1.82g, 13 mmol), and diethylene glycol was heated to 90°C and hydrazine hydrate (3.96g, 66 mmol) was added during 10 min. The mixture was further heated to 210°C for 20 min. After the mixture was cooled to room temperature, it was diluted with hexane and the organic phase was washed with water and brine, dried (MgSO₄) and concentrated in vacuo. The residue was distilled bulb to bulb to yield 2.72g (43%) of a colorless oil.

¹H-NMR (CDCl₃, 400MHz): 7.17-7.15 (m, J = 6.5 Hz, 2H), 7.05-7.03 (m, J = 6.5 Hz, 2H), 2.63 (d, J = 7.6 Hz, 2H), 2.56-2.46 (m, 1H), 2.42 (s, 3H), 2.05-1.97 (m, 2H), 1.87-1.78 (m, 2H), 1.73-1.64 (m, 2H) ppm. GC/MS (EI): 192 (M⁺, 59), 164 (33), 137 (100), 122 (18), 117 (38), 115 (24), 91 (15), 78 (6), 55 (8). IR (atr): 2969m, 2921m, 2858w, 1493s, 1438m, 1097m, 832m, 802m cm⁻¹.

Odor description: floral, sassafras, cinnamic, sweet

### Example 4: 1-Cyclopropylmethyl-4-methylsulfanyl-benzene

Synthesized analogously to the procedure of Example 3.

¹H-NMR (CDCl₃, 400MHz): 7.21-7.15 (m, 4H), 2.48 (d, J = 6.8 Hz, 2H), 2.44 (s, 3H), 0.99-0.89 (m, 1 H), 0.56-0.44 (m, 2H), 0.23-0.12 (m, 2H) ppm. GC/MS (EI): 178 (M⁺, 57), 150 (28), 137 (100), 122 (21), 91 (21), 78 (10), 63 (6). IR (atr): 3075w, 3000w, 2919w, 1493m, 1016m, 814s, 651w cm⁻¹.

Odor description: fresh, sassafras, tarragon, agrestic

### Example 5: 1-Cyclopentvlmethyl-4-methylsulfanyl-benzene

Synthesized analogously to the procedure of Example 3.

¹H-NMR (CDCl₃, 400MHz): 7.19-7.17 (m, J = 6.5 Hz, 2H), 7.10-7.08 (m, *J* = 6.5 Hz, 2H), 2.56 (d, J = 7.6 Hz, 2H), 2.46 (s, 3H), 2.11-1.99 (m, 1H), 1.73-1.47 (m, 6H), 1.22-1.13 (m, 2H) ppm. GC/MS (El): 206 (M⁺, 25), 137 (100), 122 (11), 91 (6), 78 (3), 41 (7). IR (atr): 2948s, 2919m, 2865m, 1493s, 1438m, 1097m, 967m, 829m, 794m cm⁻¹.

Odor description: floral, sassafras, cinnamic, sweet.

### Example 6

A woody, spicy fragrance was made with the following ingredients

| | parts per weight |
|---|---|
| Linalyl acetate | 20.0 |
| Ambrettolide^{™} | 6.0 |
| Bois de Gaiac ess. Paraguay | 8.0 |
| Ethylene brassylate | 200.0 |
| Calone^{™} 10 % in DPG | 3.0 |
| Cardamome grains ess. | 3.0 |
| Cashmeran^{™} | 1.0 |
| Cassis base 345 FH | 8.0 |
| Citron ess. | 35.0 |
| Cyclohexal | 50.0 |
| Dihydro myrcenol | 70.0 |
| Dipropylene glycol | 167.0 |
| Fixobois 66606 B | 25.0 |
| Galaxolide^{™} 50 PHT | 150.0 |
| ISO E Super^{™} | 95.0 |
| Lavander ess. Barreme type | 7.0 |
| Linalool synth. | 10.0 |
| Mandarine Italie Orpure | 5.0 |
| Nirvanolide^{™} | 13.0 |
| Nutmeg ess. Indonesia | 7.0 |
| Pyralone 10% in DPG | 2.0 |
| Thibetolide^{™} | 75.0 |
| Velvione^{™} | 40.0 |
| 1-Cyclopropylmethyl-4-methylsulfanyl-benzene | 3.0 |
| | 1000 |

In this woody spicy fragrance, 1-cyclopropylmethyl-4-methylsulfanyl-benzene blends excellently with the fresh spicy and woody notes of the perfume. The compound imparts a full body spicy impression to the top note but also gives freshness to the spicy woody notes of the dry down.

## Claims

1. Use of a compound of formula I as flavour or fragrance 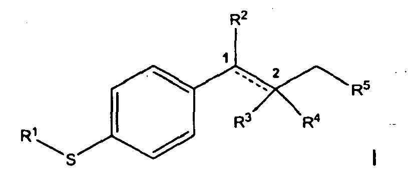 wherein
i) the bond between C₁ and C₂ is a single bond;
R¹ is methyl, ethyl, *i*-propyl, n-propyl;
R² and R³ are independently hydrogen or methyl; or
R² and R³ taken together is a divalent radical (CH₂)ₙ, C(CH₃)₂, or CH(CH₃) which forms a cycloalkane ring together with the carbon atoms to which it is attached;
R⁴ and R⁵ are independently hydrogen or methyl; or
R⁴ and R⁵ taken together is a divalent radical (CH₂)ₙ, (CH₂)ₙ₋₁CH(CH₃)₂, or (CH₂)ₙ₋₁CH(CH₃) which forms a cycloalkane ring together with the carbon atoms to which it is attached;
n is an integer of 1, 2, 3, or 4; and
wherein at least one cycloalkane ring is present; or
ii) the bond between C₁ and C₂ together with the dotted line represents a double bond;
R¹ is methyl, ethyl, *i*-propyl, n-propyl;
R² is hydrogen or methyl;
R³ and R⁴ together is hydrogen or methyl; and
R⁵ is hydrogen, methyl, ethyl, *i*-propyl, n-propyl, *n*-butyl, *tert*-butyl or *sec*-butyl.

2. Use of a compound according to claim 1 selected from the group consisting of 1-cyclopropylmethyl-4-methylsulfanyl-benzene, 1-cyclobutylmethyl-4-methylsulfanyl-benzene, 1-cyclopentylmethyl-4-methylsulfanyl-benzene, 1-cyclohexylmethyl-4-methylsulfanyl-benzene, 1-prop-1-enyl-4-methylsulfanyl-benzene and 1-hex-1-enyl-4-methylsulfanyl-benzene.

3. The use of a compound according to claim 1 to 2 in a fragrance or flavour composition.

4. A fragrance application comprising a compound as defined in any of the claims 1 to 2, or a mixture thereof.

5. A fragrance application according to claim 4 wherein the fragrance application is a perfume, household product, laundry product, body care product or cosmetic product.

6. A flavour application comprising a compound as defined in any of the claims 1 to 2, or a mixture thereof.

7. A method of flavouring or fragrancing of a product by adding one or more compounds according to one of the claims 1 to 2 to said product.

8. A compound of formula I 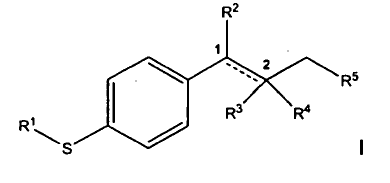 wherein
i) the bond between C₁ and C₂ is a single bond;
R¹ is methyl, ethyl, *i*-propyl, n-propyl;
R² and R³ are independently hydrogen or methyl; or
R² and R³ taken together is a divalent radical (CH₂)ₙ, C(CH₃)₂, or CH(CH₃) which forms a cycloalkane ring together with the carbon atoms to which it is attached;
R⁴ and R⁵ are independently hydrogen or methyl; or
R⁴ and R⁵ taken together is a divalent radical (CH₂)ₙ, (CH₂)ₙ₋₁CH(CH₃)₂, or (CH₂)ₙ₋₁CH(CH₃) which forms a cycloalkane ring together with the carbon atoms to which it is attached;
n is an integer of 1, 2, 3, or 4; and
wherein at least one cycloalkane ring is present; or
ii) the bond between C₁ and C₂ together with the dotted line represents a double bond;
R¹ is methyl, ethyl, *i*-propyl, *n*-propyl;
R² is hydrogen or methyl;
R³ and R⁴ together is hydrogen or methyl; and
R⁵ is hydrogen, methyl, ethyl, *i*-propyl, n-propyl, n-butyl, *tert*-butyl or *sec*-butyl, with the proviso that
if R¹ is ethyl; R² is hydrogen; and R³ and R⁴ together is hydrogen; then R⁵ is not hydrogen; or
if R¹ is methyl; R² is hydrogen; and R³ and R⁴ together is hydrogen; then R⁵ is not hydrogen or methyl.

9. A compound according to claim 8 selected from the group consisting of 1-cyclopropylmethyl-4-methylsulfanyl-benzene, 1-cyclobutylmethyl-4-methylsulfanyl-benzene, 1-cyclopentylmethyl-4-methylsulfanyl-benzene, 1-cyclohexylmethyl-4-methylsulfanyl-benzene and 1-hex-1-enyl-4-methylsulfanyl-benzene.

## Patentansprüche

1. Verwendung einer Verbindung der Formel 1 als Aroma oder Duft 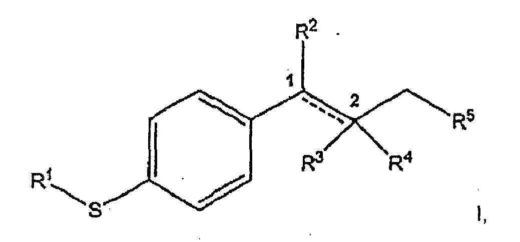 wobei
i) die Bindung zwischen C₁ und C₂ für eine Einfachbindung steht;
R¹ steht für Methyl, Ethyl, i-Propyl, n-Propyl;
R² und R³ unabhängig stehen für Wasserstoff oder Methyl; oder
R² und R³ zusammengenommen für einen zweiwertigen Rest (CH₂)ₙ, C(CH₃)₂ oder CH(CH₃) steht, der einen Cycloalkanring bildet zusammen mit den Kohlenstoffatomen, an die er gebunden ist;
R⁴ und R⁵ unabhängig stehen für Wasserstoff oder Methyl; oder
R⁴ und R⁵ zusammengenommen für einen zweiwertigen Rest (CH₂)ₙ, (CH₂)ₙ₋₁CH(CH₃)₂ oder (CH₂)ₙ₋₁CH(CH₃) steht, der einen Cycloalkanring bildet zusammen mit den Kohlenstoffatomen, an die er gebunden ist;
n für eine ganze Zahl von 1, 2, 3 oder 4 steht; und
wobei mindestens ein Cycloalkanring vorliegt; oder
(ii) die Bindung zwischen C₁ und C₂ zusammen mit der gepunkteten Linie für eine Doppelbindung steht;
R¹ steht für Methyl, Ethyl, i-Propyl, n-Propyl;
R² steht für Wasserstoff oder Methyl;
R³ und R⁴ zusammen steht für Wasserstoff oder Methyl; und
R⁵ steht für Wasserstoff, Methyl, Ethyl, i-Propyl, n-Propyl, n-Butyl, tert.-Butyl oder sek.-Butyl.

2. Verwendung einer Verbindung nach Anspruch 1, die ausgewählt ist aus der Gruppe, bestehend aus 1-Cyclopropylmethyl-4-methylsulfanylbenzol, 1-Cyclobutylmethyl-4-methylsulfanylbenzol, 1-Cyclopentylmethyl-4-methylsulfanylbenzol, 1-Cyclohexylmethyl-4-methylsulfanylbenzol, 1-Prop-1-enyl-4-methylsulfanylbenzol und 1-Hex-1-enyl-4-methylsulfanylbenzol.

3. Verwendung einer Verbindung nach Anspruch 1 oder 2 in einer Duft- oder Aromazusammensetzung.

4. Duftanwendung, umfassend eine Verbindung, wie sie in einem der Ansprüche 1 bis 2 definiert ist, oder ein Gemisch davon.

5. Duftanwendung nach Anspruch 4, wobei die Duftanwendung für ein Parfüm, Haushaltsprodukt, Wäscheprodukt, Körperpflegeprodukt oder Kosmetikprodukt steht.

6. Aromaanwendung, umfassend eine Verbindung, wie sie in einem der Ansprüche 1 bis 2 definiert ist, oder ein Gemisch davon.

7. Verfahren eines Aromatisierens eines Produkts oder eines Versehens eines Produkts mit einem Duft durch Zugeben einer oder mehrerer Verbindung(en) nach einem der Ansprüche 1 bis 2 zu dem Produkt.

8. Verbindung der Formel I 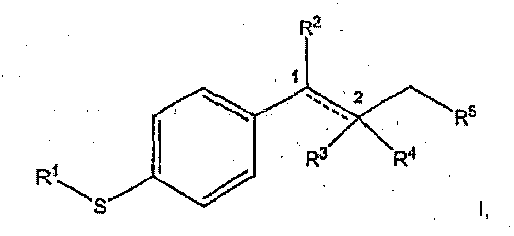 wobei
i) die Bindung zwischen C₁ und C₂ für eine Einfachbindung steht;
R¹ steht für Methyl, Ethyl, 1-Propyl, n-Propyl;
R² und R³ unabhängig stehen für Wasserstoff oder Methyl; oder
R² und R³ zusammengenommen für einen zweiwertigen Rest (CH₂)ₙ, C(CH₃)₂ oder CH(CH₃) steht, der einen Cycloalkanring bildet zusammen mit den Kohlenstoffatomen, an die er gebunden ist;
R⁴ und R⁵ unabhängig stehen für Wasserstoff oder Methyl; oder
R⁴ und R⁵ zusammengenommen für einen zweiwertigen Rest (CH₂)ₙ, (CH₂)ₙ₋₁CH(CH₃)₂ oder (CH₂)ₙ₋₁CH(CH₃) steht, der einen Cycloalkanring bildet zusammen mit den Kohlenstoffatomen, an die er gebunden ist;
n für eine ganze Zahl von 1, 2, 3 oder 4 steht; und
wobei mindestens ein Cycloalkanring vorliegt; oder
(ii) die Bindung zwischen C₁ und C₂ zusammen mit der gepunkteten Linie für eine Doppelbindung steht;
R¹ steht für Methyl, Ethyl, i-Propyl, n-Propyl;
R² steht für Wasserstoff oder Methyl;
R³ und R⁴ zusammen steht für Wasserstoff oder Methyl; und
R⁵ steht für Wasserstoff, Methyl, Ethyl, i-Propyl, n-Propyl, n-Butyl, tert.-Butyl oder sek.-Butyl, mit der Maßgabe, dass
wenn R¹ für Ethyl steht; R² für Wasserstoff steht; und R³ und R⁴ zusammen für Wasserstoff steht; dann R⁵ nicht für Wasserstoff steht; oder
wenn R¹ für Methyl steht; R² für Wasserstoff steht; und R³ und R⁴ zusammen für Wasserstoff steht; dann R⁵ nicht für Wasserstoff oder Methyl steht.

9. Verbindung nach Anspruch 8, ausgewählt aus der Gruppe, bestehend aus 1-Cyclopropylmethyl-4-methylsulfanylbenzol, 1-Cyclobutylmethyl-4-methylsulfanylbenzol, 1-Cyclopentylmethyl-4-methylsulfanylbenzol, 1-Cyclohexylmethyl-4-methylsulfanylbenzol und 1-Hex-1-enyl-4-methylsulfanylbenzol.

## Revendications

1. Utilisation d'un composé de formule I en tant qu'arôme ou parfum 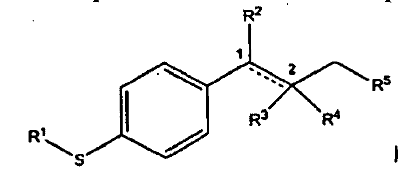 dans laquelle
i) la liaison entre C₁ et C₂ est une liaison simple ;
R¹ est le groupe méthyle, éthyle, isopropyle, n-propyle ;
R² et R³ représentent chacun indépendamment de l'autre un atome d'hydrogène ou le groupe méthyle ; ou
R² et R³, pris ensemble, forment un radical divalent (CH₂)ₙ, C(CH₃)₂ ou CH(CH₃), qui forme un noyau cycloalcane avec les atomes de carbone auxquels il est lié ;
R⁴ et R⁵ représentent chacun indépendamment de l'autre un atome d'hydrogène ou le groupe méthyle, ou
R⁴ et R⁵, pris ensemble, forment un radical divalent (CH₂)ₙ, (CH₂)ₙ₋₁CH(CH₃)₂ ou (CH₂)ₙ₋₁CH(CH₃), qui forme un noyau cycloalcane avec les atomes de carbone auxquels il est lié ;
n est un entier valant 1, 2, 3 ou 4 ; et
dans lequel au moins un noyau cycloalcane est présent ; ou bien
ii) la liaison entre C₁ et C₂, avec la ligne en tirets, représente une double liaison ;
R¹ est le groupe méthyle, éthyle, isopropyle, n-propyle ;
R² est un atome d'hydrogène ou le groupe méthyle ;
R³ et R⁴ forment ensemble un atome d'hydrogène ou un groupe méthyle ; et
R⁵ est un atome d'hydrogène, ou le groupe méthyle, éthyle, isopropyle, n-propyle, n-butyle, tert-butyle ou sec-butyle.

2. Utilisation d'un composé selon la revendication 1, choisi dans le groupe consistant en le 1-cyclopropylméthyl-4-méthylsulfanyl-benzène, le 1-cyclobutylméthyl-4-méthylsulfanyl-benzène, le 1-cyclopentylméthyl-4-méthylsulfanyl-benzène, le 1-cyclohexylméthyl-4-méthylsulfanyl-benzène, le 1-prop-1-ényl-4-méthylsulfanyl-benzène et le 1-hex-1-ényl-4-méthylsulfanyl-benzène.

3. Utilisation d'un composé selon la revendication 1 ou 2 dans une composition d'arôme ou de parfum.

4. Application d'un parfum comprenant un composé selon l'une quelconque des revendications 1 ou 2, ou un mélange de ceux-ci.

5. Application d'un parfum selon la revendication 4, où l'application du parfum est un parfum, un produit ménager, un produit pour le blanchissage, un produit pour les soins corporels ou un produit cosmétique.

6. Application d'un arôme comprenant un composé selon l'une quelconque des revendications 1 ou 2, ou un mélange de ceux-ci.

7. Composé pour aromatiser ou parfumer un produit par addition, audit produit, d'un ou plusieurs composés selon l'une des revendications 1 ou 2.

8. Composé de formule I 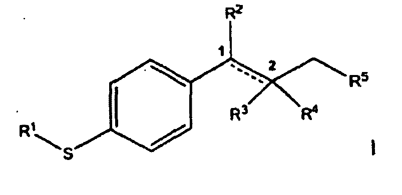 dans laquelle
i) la liaison entre C₁ et C₂ est une liaison simple ;
R¹ est le groupe méthyle, éthyle, isopropyle, n-propyle ;
R² et R³ représentent chacun indépendamment de l'autre un atome d'hydrogène ou le groupe méthyle ; ou
R² et R³, pris ensemble, forment un radical divalent (CH₂)ₙ, C(CH₃)₂ ou CH(CH₃), qui forme un noyau cycloalcane avec les atomes de carbone auxquels il est lié ;
R⁴ et R⁵ représentent chacun indépendamment de l'autre un atome d'hydrogène ou le groupe méthyle, ou
R⁴ et R⁵, pris ensemble, forment un radical divalent (CH₂)ₙ, (CH₂)ₙ₋₁CH(CH₃)₂ ou (CH₂)ₙ₋₁CH(CH₃), qui forme un noyau cycloalcane avec les atomes de carbone auxquels il est lié ;
n est un entier valant 1, 2, 3 ou 4 ; et
dans lequel au moins un noyau cycloalcane est présent ; ou bien
ii) la liaison entre C₁ et C₂, avec la ligne en tirets, représente une double liaison ;
R¹ est le groupe méthyle, éthyle, isopropyle, n-propyle ;
R² est un atome d'hydrogène ou le groupe méthyle ;
R³ et R⁴ forment ensemble un atome d'hydrogène ou un groupe méthyle ; et
R⁵ est un atome d'hydrogène, ou le groupe méthyle, éthyle, isopropyle, n-propyle, n-butyle, tert-butyle ou sec-butyle,
à la condition que :
si R¹ est le groupe éthyle, R² est un atome d'hydrogène et R³ et R⁴ forment ensemble un atome d'hydrogène, alors R⁵ ne soit pas un atome d'hydrogène ; ou bien
si R¹ est le groupe méthyle, R² est un atome d'hydrogène et R³ et R⁴ forment ensemble un atome d'hydrogène, alors R⁵ ne soit pas un atome d'hydrogène ou le groupe méthyle.

9. Composé selon la revendication 8, choisi dans le groupe consistant en le 1-cyclopropylméthyl-4-méthylsulfanyl-benzène, le 1-cyclobutylméthyl-4-méthylsulfanyl-benzène, le 1-cyclopentylméthyl-4-méthylsulfanyl-benzène, le 1-cyclohexylméthyl-4-méthylsulfanyl-benzène et le 1-hex-1-ényl-4-méthylsulfanyl-benzène.
